# FASCICULE DE BREVET EUROPEEN

(11) **EP 1 056 477 B1**
(45) Date de publication et mention de la délivrance du brevet: **07.12.2005**
(21) Numéro de dépôt: 99906300.1
(22) Date de dépôt: 24.02.1999
(51) Int. Cl.: A61K 47/48, A61K 9/51

(54) **NANOPARTICULES COMPRENANT POLYISOBUTYLCYANOACRYLATE ET CYCLODEXTRINES**
NANOPARTIKEL DIE POLYISOBUTYLCYANOACRYLATE UND CYCLODEXTRINE ENTHALTEN
NANOPARTICLES COMPRISING POLYISOBUTYLCYANOACRYLATE AND CYCLODEXTRINS

(30) Priorité: 27.02.1998 FR 9802429
(43) Date de publication de la demande: 06.12.2000
(73) Titulaire: Bioalliance Pharma (S.A.), 75013 Paris (FR)
(72) Inventeur: MONZA DA SILVEIRA, Airton, CEP-91720-110 Porto Alegre, RS (BR); PONCHEL, Gilles, F-75012 Paris (FR); DUCHENE, Dominique, F-75116 Paris (FR); COUVREUR, Patrick, F-91140 Villebon sur Yvette (FR); PUISIEUX, Francis, F-94700 Maisons Alfort (FR)
(74) Mandataire: Breese, Pierre
(86) Numéro de dépôt international: PCT/FR1999/000418
(87) Numéro de publication internationale: WO 1999/043359

(56) Documents cités:
- WO-A-92/04916
- WO-A-93/25195
- US-A- 4 913 908
- US-A- 5 641 515
- MONZA DA SILVEIRA A ET AL: "Combined poly(isobutylcyanoacrylate) and cyclodextrins nanoparticles for enhancing the encapsulation of lipophilic drugs." PHARM RES, JUL 1998, 15 (7) P1051-5, XP002085312 UNITED STATES
- EGEA, MARIA ANTONIA ET AL: "Entrapment of cisplatin into biodegradable poly(alkyl cyanoacrylate) nanoparticles" FARMACO, 1994, 49, 211-17, XP002085314
- BAPAT, NITEEN ET AL: "Uptake capacity and adsorption isotherms of doxorubicin on polymeric nanoparticles: effect of methods of preparation" DRUG DEV. IND. PHARM., 1992, 18, 65-77, XP002085319
- FATTAL E ET AL: "Biodegradable polyalkylcyanoacrylate nanoparticles for the delivery of oligonucleotides" JOURNAL OF CONTROLLED RELEASE, vol. 53, no. 1-3, 30 avril 1998, page 137-143 XP004121264
- COUVREUR P.: "Polyalkylcyanoacrylates as colloidal drug carriers" CRC CRIT. REV. THER. DRUG CARRIER SYST., 1988, 5/1 (1-20), XP002085327 USA
- SALINAS, C ET AL: "Coadjuvant effect in Combinations of carboplatin and poly(alkyl cyanoacrylate)." CIENC. PHARM., 1993, 3, 253-62, XP002085318
- GIBAUD S ET AL: "Polyalkylcyanoacrylate nanoparticles as carriers for granulocyte -colony stimulating factor (G-CSF)" JOURNAL OF CONTROLLED RELEASE, vol. 52, no. 1-2, 2 mars 1998, page 131-139 XP004113661
- DUCHENE DOMINIQUE_(A): "Cyclodextrins in targeting: Application to nanoparticles." ADVANCED DRUG DELIVERY REVIEWS, 1999, XP002104026

## Description

L'invention concerne la délivrance de principes actifs utilisés notamment dans le domaine des médicaments à visée préventive, curative ou diagnostique et également l'amélioration de leur index thérapeutique (amélioration du rapport bénéfices/risques).
Elle a plus particulièrement pour objet de nouvelles nanoparticules renfermant au moins un principe actif.
La mise au point de nouveaux systèmes de délivrance ou libération de principes actifs a pour objectif premier la délivrance contrôlée d'un agent actif, notamment pharmacologique, à son site d'action à une vitesse et à une posologie thérapeutiquement optimales (1). L'amélioration de l'index thérapeutique peut être obtenue par modulation de la distribution du principe actif dans l'organisme. L'association du principe actif au système de délivrance permet notamment sa délivrance spécifiquement au site d'action ou sa libération contrôlée après le ciblage du site d'action. La réduction de la quantité de principe actif dans les compartiments où sa présence n'est pas souhaitable permet d'accroître l'efficacité dudit principe actif, de réduire ses effets secondaires toxiques, voire même de modifier ou de restaurer son activité.

Les systèmes colloïdaux de délivrance de principes actifs comprennent les liposomes, les microémulsions, les nanocapsules, les nanosphères, les microparticules et les nanoparticules. Les nanoparticules présentent des avantages de ciblage, de modulation de distribution et de souplesse de formulation et ont une structure polymère qui peut être conçue et réalisée de façon adaptée au but poursuivi. Elles se sont révélées tout particulièrement prometteuses pour obtenir un meilleur index thérapeutique au sens défini ci-dessus, en raison de leur aptitude à assurer une libération contrôlée, une délivrance spécifique au site d'action ou délivrance ciblée, permettant à la fois une augmentation de l'efficacité et une réduction des effets secondaires toxiques au niveau des autres organes.
Ce type d'administration nécessite l'emploi de polymères biodégradables. Parmi ceux-ci, les poly(cyanoacrylates d'alkyle) sont particulièrement intéressants car leur bioérosion est observée rapidement par rapport à d'autres polymères biodégradables et se déroule pendant des durées compatibles avec les applications thérapeutiques ou diagnostiques.
Malgré ces caractéristiques intéressantes, la capacité de charge en principes actifs des nanoparticules de poly(cyanoacrylates d'alkyle), exprimée en tant que quantité de principe actif associée à une unité de masse de polymère, est souvent limitée, notamment lorsque le principe actif est très faiblement soluble dans l'eau car la fabrication des nanoparticules utilise des techniques de polymérisation en milieu aqueux. Cette limitation importante de la charge en principe actif s'observe en particulier avec les principes actifs hydrophobes, amphiphiles et/ou insolubles.
L'aptitude relativement faible des nanoparticules conventionelles à transporter une quantité adéquate de principes actifs du site d'administration au site cible dans l'organisme risque souvent de conduire à la nécessité d'administrer des quantités considérables de polymères.

Les poly(cyanoacrylates d'alkyle) sont utilisés pour produire des nanoparticules en tant que vecteurs de principes actifs (3). Toutefois, pour les raisons évoquées ci-dessus, les faibles charges obtenues, notamment avec les principes actifs hydrophobes, amphiphiles et/ou insolubles dans l'eau en limitent l'usage thérapeutique.

On a maintenant trouvé de façon surprenante qu'il était possible d'élargir le domaine d'utilisation des polymères, en particulier des poly(cyanoacrylates d'alkyle), en leur associant un ou plusieurs composés aptes à complexer des principes actifs et ainsi obtenir de nouvelles nanoparticules possédant des propriétés originales.
L'invention a donc pour objet des nanoparticules contenant au moins un principe actif, caractérisées en ce qu'elles comprennent l'association d'au moins un polymère, de préférence un poly(cyanoacrylate d'alkyle) dans lequel le groupe alkyle, linéaire ou ramifié, comprend de 1 à 12 atomes de carbone, et d'au moins un composé apte à complexer ledit principe actif.

Le composé apte à complexer le principe actif selon la présente invention est de préférence choisi parmi les oligosaccharides cycliques, notamment parmi les cyclodextrines qui peuvent être neutres ou chargées, natives (cyclodextrines α, β, γ, δ, ε), branchées ou polymérisées ou encore modifiées chimiquement par exemple par substitution d'un ou plusieurs hydroxypropyles par des groupements tels que alkyles, aryles, arylalkyles, glycosidiques, ou par etherification, estérification avec des alcools ou des acides aliphatiques. Parmi les groupements ci-dessus, on préfère plus particulièrement ceux choisis parmi les groupements hydroxypropyle, méthyle, sulfobutylether.
De façon inattendue, la présence d'un composé apte à complexer le principe actif dans l'association selon l'invention permet au principe actif, même s'il est hydrophobe, amphiphile et/ou insoluble, de pénétrer à l'intérieur de la structure polymérique résultant de l'association du ou des polymères et du ou desdits composés aptes à complexer le principe actif, et ceci avec un rendement d'encapsulation dans cette structure significativement accru par rapport à l'art antérieur, rendement qui semble lié à l'équilibre entre d'une part, la solubilisation résultant de l'utilisation de composés aptes à complexer le principe actif et, d'autre part, l'affinité du principe actif pour la nouvelle structure polymérique, ce qui constitue un progrès important sur les plans thérapeutique et industriel. Par ailleurs, les nanoparticules stabilisent également le complexe formé entre le(s)dit(s) composé (s) et le(s)dit(s) polymère(s) en raison de la nature solide des nanoparticules.
Grâce à l'invention, il est maintenant possible de charger des nanoparticules par exemple de type poly(cyanoacrylate d'alkyle) non seulement avec les principes actifs hydrophiles mais également les principes actifs hydrophobes, amphiphiles et/ou insolubles.
L'association d'un polymère et d'un composé apte à complexer le principe actif permet la création de nouveaux sites de fixation pour le principe actif qui n'apparaissent pas avec les polymères utilisés seuls. L'apparition de ces sites nouveaux, et en particulier celle d'une cavité hydrophobe avec les composés aptes à complexer les principes actifs, permet d'augmenter la charge en principe actif tout en maintenant la capacité de libération contrôlée et retardée de celui-ci qui est inexistante lorsqu'on utilise seuls les composés aptes à complexer.

On a décrit dans l'art antérieur la préparation de polymères à base de cyanoacrylate, où les alkylcyanoacrylates sont associés au dextran au cours de la préparation (Egea, M. A. et al., Farmaco, 1994, 49, 211-17) Or, dans cette méthode le dextran est utilisé classiquement en tant qu'agent stabilisant et ne permet pas de complexer une molécule active. En outre, le dextran est un polysaccharide linéaire de masse moléculaire élevée et il est donc fondamentalement différent des cyclodextrines qui présente un faible poids moléculaire et qui sont capables de complexer d'autres molécules. Ainsi, les nanoparticules selon la présente invention présentent des propriétés originales :
- modulation de leur taille,
- encapsulation augmentée de molécules actives, notamment des molécules , hydrophobes, amphiphiles et/ou insolubles,
- absence éventuelle de stabilisant tel que le dextran.
Il a également été proposé dans le brevet US No. 5 641 515 d'encapsuler l'insuline avec un polymère de polycyanoacrylate. Cette encapsulation est basée sur la formation de liaisons covalentes entre l'insuline et le polymère ce qui est différent de la complexation à la base des nanoparticules selon la présente invention. En effet, les nanoparticules selon l'invention sont fondées la capacité d'une molécule d'un principe actif de s'associer avec une, ou plusieurs, molécule de cyclodextrine par la création de liaisons chimiques de faibles énergie ,donc non covalentes, de façon à former un complexe d'inclusion. L'existence de ce complexe résulte de la formation d'un équilibre entre, d'une part, les formes libres du principe actif et de la cyclodextrine et d'autre part, du complexe d'inclusion. Il est quantitativement caractérisé par sa constante de stabilité. Au sens de la présente invention, le terme complexation décrit exclusivement ce dernier phénomène. Ainsi, la complexation du principe actif est mise en oeuvre non seulement au cours de la préparation des nanoparticules mais également dans les nanoparticules préparées, où elle représente un moyen d'associer une quantité plus importante de principe actif.
Il convient en effet de rappeler que de manière générale, l'association d'un principe actif à des nanoparticules peut résulter d'une simple dispersion du principe actif sous forme de cristaux dans le polymère constitutif des particules, d'une solubilité du principe actif dans le polymère, d'une adsorption faisant intervenir des liaisons chimiques secondaires (faibles énergies), ou enfin d'une liaison covalente (forte énergie) avec le polymère constitutif des particules.
A cet égard, il est utile d'indiquer que la préparation de nanoparticules nécessite la polymérisation des monomères de cyanoacrylate d'alkyles dispersés en phase aqueuse. La synthèse du poly(cyanoacrylate d'alkyle) permet alors la formation des nanoparticules. Généralement, cette étape est conduite en présence des principes actifs à encapsuler. Elle peut donc avoir comme conséquence, dans certains cas, le développement non souhaité de liaisons chimiques covalentes entre le principe actif et le polymère formé. Ce phénomème a été décrit pour des peptides (Grangier, J. l., J; Controlled Rel., 15, 3-13, 1991) ou d'autres molécules (vinblastine, V. Guise et al., Pharm. Res., 7, 736-741, 1990).
La présente invention permet de palier cet inconvénient, car en masquant les groupements chimiques potentiellement réactifs, la complexation du principe actif au cours de la préparation des nanoparticules de l'invention permet de protéger ledit principe actif vis-à-vis des réactions chimiques nécessaires à la formation de la particule. Ainsi, le principe actif est avantageusement associé de manière non covalente à la particule.
En outre, l'association du principe actif aux nanoparticules s'effectue généralement dans un milieu aqueux acide. Or, pour certains principes actifs instables dans ces conditions, il en résulte un risque de dégradation chimique susceptible d'aboutir à l'encapsulation non souhaitée de dérivés d'hydrolyse et, de plus, préjudiciable à l'obtention d'un taux d'encapsulation élevé du principe actif. En revanche, dans la présente invention, la complexation des principes actifs aux cyclodextrines permet de palier ces inconvénients car elle permet de protéger les principes actifs vis-à-vis du milieu réactionnel extérieur.

A titre de principes actifs susceptibles d'entrer dans la composition des nanoparticles de l'invention, on peut citer les anticancéreux, les antisens, les antiviraux, les antibiotiques, les protéines, polypeptides, polynucléotides, nucléotides antisens, les substances vaccinantes, les immunomodulateurs, les stéroïdes, les analgésiques, les antimorphiniques, les antifongiques et antiparasitaires. Parmi ceux-ci, l'invention envisage tout particulièrement le taxol ou l'un de ses dérivés, la doxorubicine ou l'un de ses dérivés, les dérivés du platine.

Le principe actif est en général présent en une quantité de 0,01 à 300 mg/g de nanoparticules.
La proportion de composé apte à complexer le principe actif est en général de 0,1 à 70 % en poids.

La proportion de principe actif et celle du composé apte à complexer sont indépendantes l'une de l'autre.

L'invention concerne aussi bien entendu les compositions pharmaceutique ou de diagnostic comprenant les nanoparticules de l'invention et au moins un véhicule pharmaceutiquement acceptable et compatible.

L'invention a également pour objet la préparation des nanoparticules décrites précédemment.
Un premier procédé de préparation des nanoparticules à base d'un polymère et plus particulièrement de poly(cyanoacrylate d'alkyle) définies précédemment, est caractérisé en ce qu'il comprend les étapes consistant à :
a) préparer un complexe d'au moins un principe actif avec au moins un composé apte à le complexer, en solution dans un solvant aqueux ou non aqueux,
b) ajouter progressivement au moins un monomère du polymère, et plus particulièrement le cyanoacrylate d'alkyle monomère dans la solution obtenue à l'étape (a), et
c) effectuer une polymérisation de préférence anionique mais également inductible par d'autres agents notamment photochimiques de ce monomère, éventuellement en présence d'un ou plusieurs agents tensio-actif et/ou stabilisant.

Un second procédé de préparation des nanoparticules selon l'invention, constituant une alternative au premier procédé ci-dessus, consiste à préparer d'abord des nanoparticules à base d'un polymère et plus particulièrement des poly(cyanoacrylate d'alkyle) et d'un composé apte à complexer un principe actif, désignées aussi "nanoparticules blanches" puis à associer auxdites nanoparticules blanches le principe actif. Plus particulièrement, ce procédé comprend les étapes consistant à :
a) préparer une solution d'au moins un composé apte à complexer un pricipe actif dans un solvant aqueux ou non aqueux,
b) ajouter progressivement au moins un monomère du polymère, et plus particulièrement le cyanoacrylate d'alkyle monomère dans la solution de l'étape (a), et
c) effectuer une polymérisation de préférence anionique mais également inductible par d'autres agents notamment photochimiques de ce monomère, éventuellement en présence d'un ou plusieurs agents tensio-actif et/ou stabilisant,
d) après un contrôle et une prufification éventuelle des nanoparticules obtenus à l'étape (c), incuber lesdites particules dans une solution du principe actif dans un solvant aqueux ou non aqueux.
Comme dans le premier procédé, l'association du principe actif aux nanoparticules blanches dépendra de la quantité de cyclodextrines associée aux nanoparticules Ce second procédé présente deux avantages :
- il permet d'éviter d'avoir à effectuer les étapes de purification sur les nanoparticules chargées en principe actif, celle-ci pouvant aboutir à des pertes de principe actif,
- il permet de réaliser un système susceptible d'être chargé extemporanément en principe actif, par exemple dans le cas où un principe actif est très instable en solution.
L'invention se rapporte donc également aux nanoparticules blanches, c'est à dire non chargées, obtenues après les étapes (a) à (c) du second procédé décrit ci-dessus. En outre, ces particules blanches présentent un intérêt thérapeutique du fait de l'activité des cyclodextrines notamment dans le domaine du cancer (Grosse, P. Y. et al, British Journal of Cancer, 78 : 9, 1165-1169, 1998).

Aux étapes (a) et (b) du premier procédé de l'invention, le solvant est avantageusement choisi de façon à ce que, tout en maintenant des conditions favorables à la polymérisation des polymères et plus particulièrement des poly(cyanoacrylate d'alkyle), la solubilité du principe actif et du composé apte à le complexer soit maximale dans le milieu défini par ce solvant. Avantageusement un tel solvant est choisi de préférence parmi les solvants aqueux ou hydroalcooliques. Le solvant est choisi de la façon aux étapes (a), (b) et (d) du second procédé de l'invention.

La présence d'un agent tensio-actif ou d'un agent stabilisant est nécessaire pour préparer les nanoparticules de l'art antérieur. Comme le montrent les exemples qui suivent, de tels agents ne sont plus nécessaires dans le cadre de la présente invention. En effet, le composé apte à complexer le principe actif, comme les cyclodextrines, ont paradoxalement un effet stabilisant suffisant pour que l'agent tensio-actif habituellement utilisé soit absent. Ceci représente, sur le plan industriel, une économie notable. De même, on observe que le poly(cyanoacrylate d'alkyle) stabilise le complexe formé du principe actif et du composé apte à complexer le principe actif.
Cependant, si le procédé de l'invention comprend l'utilisation d'un agent stabilisant et/ou tensio-actif on préfère un dextran, ou un poloxamer.

Selon une forme de réalisation préférée de l'invention, les potentialités des cyclodextrines vis-à-vis des principes actifs permettent d'adjoindre de nouvelles propriétés aux particules. En effet, la présence des cyclodextrines dans les particules permet de stabiliser les principes actifs qui seraient instables en solution ou encore de masquer certaines caractéristiques défarobles des principes actifs telle qu'une action irritante.

Les procédés de fabrication des nanoparticules connus jusqu'à ce jour présentent des lacunes en ce qui concerne les possibilités d'ajustement de la taille des nanoparticules. Le procédé de l'invention permet de façon inattendue et remarquable d'ajuster la taille des nanoparticules directement au cours de leur fabrication sans aucune étape particulière supplémentaire.
Comme le montrent les exemples qui suivent, la taille des nanoparticules selon l'invention est essentiellement fonction de la concentration en composé apte à complexer le principe actif. Dans le cas des cyclodextrines, on peut ainsi faire varier cette taille dans une gamme très large de 300 à moins de 50 nm. L'invention permet donc, à l'aide d'essais préliminaires simples, d'ajuster la taille des nanoparticules dans les compositions, notamment pharmaceutiques, de l'invention en fonction de l'effet particulier recherché. Le choix de la taille *a priori* permet, si on le souhaite, de s'affranchir de certaines barrières physiques s'opposant à la distribution des nanoparticules l'organisme ou d'éviter une capture des nanoparticules de la composition par le système réticulo-endothélial. Il permet aussi un nouveau ciblage d'organes.

En conséquence à l'étape (a) du procédé de l'invention la proportion de composé apte à complexer le principe actif est en général de 0,1 à 70 % en poids par rapport audit principe actif. En effet, comme indiqué précédemment, le choix de la concentration du composé apte à complexer le principe actif permet de faire varier la taille des nanoparticules obtenues par le procédé de l'invention. On obtient ainsi des nanoparticules de taille comprise entre 40 et 300 nm.

Les études relatives à la libération du composé apte à complexer le principe actif, d'une part et du principe actif, d'autre part, montrent que le profil de libération du composé apte à complexer le principe actif est très rapide et que la libération est proche de 100%, tandis que la libération du principe actif comprend une première phase rapide, suivie d'une deuxième phase plus lente due à la bioérosion, classiquement décrite pour les poly (cyanoacrylates).
L'utilisation, dans les essais de libération du principe actif, d'estérases qui dégradent les nanoparticules, montre que le principe actif est contenu en grande partie au sein du réseau matriciel nanoparticulaire, ce qui est important du point de vue de l'activité attendue (4).
Les différents essais effectués sur une gamme de stéroïdes, du plus hydrophile (hydrocortisone) au plus hydrophobe (progestérone) ont montré que des principes actifs très variés peuvent être contenus dans les nanoparticules selon l'invention à des concentrations élevées, dépendantes de leurs caractéristiques physico-chimiques telles que notamment leur degré d'hydrophobicité.
Ainsi, la progestérone utilisée comme modèle dans les exemples qui suivent a une solubilité dans l'eau très faible (0,01 mg/ml) qui, dans les processus classiques de polymérisation en émulsion dans l'eau, ne permet d'obtenir qu'une charge très faible en principe actif, dépourvue d'intérêt pratique. Ainsi, cette charge est faible lorsqu'on utilise les techniques de préparation de l'art antérieur. De façon particulièrement surprenante et intéressante, cette charge est de plus de 50 fois supérieure dans les nanoparticules selon l'invention. L'invention permet donc d'accéder à des principes actifs hydrophobes, amphiphiles et/ou insolubles et donc un renouveau de leur index thérapeutique.
L'invention a donc aussi pour objet l'utilisation des procédés décrits ci-dessus pour fabriquer un médicament à effet ciblé et à index thérapeutique amélioré.

D'autres avantages et caractéristiques de l'invention apparaîtront de la description des exemples qui suivent faisant références aux dessins annexés dans lesquels :
La Figure 1 représente les variations de la taille des particules ou granulométrie et du potentiel zéta (moyenne de trois essais ± SEM) de nanoparticules de poly(cyanoacrylate d'isobutyle) (PIBCA) préparées en présence de 2-hydroxypropyl-β-cyclodextrine (HPβCD), en fonction de la concentration initiale en HPβCD.
La Figure 2 représente les variations de la granulométrie et du potentiel zéta (moyenne de trois essais ± SEM) de nanoparticules de PIBCA préparées en présence du complexe progestérone : 2-hydroxypropyl-β-cyclodextrine (HPβCD), en fonction de la concentration initiale en HPβCD.
La Figure 3 représente les variations de la teneur en HPβCD (moyenne de trois essais ± SEM) de nanoparticules de PIBCA préparées en présence de HPβCD, en fonction de la concentration initiale en HPβCD.
La Figure 4 représente les variations de la teneur en HPβCD (moyenne de trois essais ± SEM) de nanoparticules de PIBCA préparées en présence du complexe progestérone : HPβCD, en fonction de la concentration initiale en HPβCD.
La Figure 5 représente les variations de la teneur en progestérone (moyenne de trois essais ± SEM) de nanoparticules de PIBCA/HPβCD, en fonction de la concentration initiale en HPβCD.
La Figure 6 montre l'influence de la taille des particules sur la vitesse de libération de la progestérone dans du tampon au borate alcalin (ABB) (pH 8,4), à partir de nanoparticules de PIBCA/HPβCD.
La Figure 7 montre l'influence de la constitution du milieu de libération sur la vitesse de libération de la progestérone dans du milieu ABB (pH 8,4), à partir de nanoparticules de PIBCA/HPβCD.
   A : ABB:PEG 400 (80:20)
   B : ABB:PEG 400 (60:40)
La Figure 8 montre l'influence de la présence d'enzymes de type estérase sur la vitesse de libération de la progestérone dans du milieu ABB (pH 8,4), à partir de nanoparticules de PIBCA/HPβCD.
   A : milieu de libération avec de l'estérase 25 UI
   B : milieu de libération avec de l'estérase 100 UI
La Figure 9 montre la vitesse de libération de la HPβCD dans du milieu ABB à 37°C.
La Figure 10 montre les courbes de calorimétrie par analyse différentielle (DSC) obtenues à une vitesse d'accroissement de la température de 10°C/min.

Dans les exemples qui suivent, le cyanoacrylate d'isobutyle, l'hydrocortisone, la prednisolone et le danazol la progestérone et les estérases (19 UI/ml) ont été obtenus auprès de Sigma Chemicals (St. Louis, Mo, EUA), la spironolactone, la testostérone, l'acétate de mégestrol ont été obtenue respectivement auprès de Sophartex, Besin-Iscovesco et Upjohn, les α-, β- et γ-cyclodextrines, les 2-hydroxypropyl-α-, 2-hydroxypropyl-β- et 2-hydroxypropyl-γ-cyclodextrines, dont les valeurs de MS moyennes sont respectivement de 0,9, 0,6 et 0,6, ont été obtenues auprès de Wacker Chemie GmbH (Munich, Allemagne) et l'éther de sulfobutyle et de β-cyclodextrine (ci-après SBEβCD) a été obtenu auprès de CyDex L. C. (Overland Park, Kansas, EUA). Le poloxamer 188 (Lutrol F68®) est un don de BASF (Ludwigshafen, Allemagne). Les autres produits chimiques et les solvants sont de qualité analytique et HPLC.

### EXEMPLE 1 Préparation de nanoparticules en présence de différentes cyclodextrines et de poloxamer.

Les nanoparticules sont préparées par polymérisation anionique (2) de 100 µl de cyanoacrylate d'isobutyle dans 10 ml d'acide chlohydrique 0,01 M (pH 2,0) contenant 1% p/v de poloxamer 188 et en présence de 5 mg/ml de α-, β- , γ-, 2-hydroxypropyl-α-, 2-hydroxypropyl-β- ou 2-hydroxypropyl-γ-cyclodextrine ou de sulfobutyle éther de β-cyclodextrine. La solution de cyclodextrine est agitée au moyen d'un agitateur magnétique (1000 tr/min) à la température ambiante et le monomère est ajouté goutte à goutte. Après agitation pendant 6 heures, la suspension est filtrée au moyen d'un préfiltre de 2,0 µm (Millex AP 500®) puis encore caractérisée.

### EXEMPLE 2 : Préparation de nanoparticules en présence de différentes cyclodextrines.

Les nanoparticules sont préparées par polymérisation anionique (2) de 100 µl de cyanoacrylate d'isobutyle dans 10 ml d'acide chlorhydrique 0,01 M (pH 2,0) et en présence de 5 mg/ml de α-, β-, γ-, 2-hydroxypropyl-α-, 2-hydroxypropyl-β- ou 2-hydroxypropyl-γ-cyclodextrine ou de sulfobutyle éther de β-cyclodextrine. La solution de cyclodextrine est agitée au moyen d'un agitateur magnétique (1000 tr/min) à la température ambiante et le monomère est ajouté goutte à goutte. Après agitation pendant 6 heures, la suspension est filtrée au moyen d'un préfiltre de 2,0 µm (Millex AP 500®) puis encore caractérisée.

### EXEMPLE 3 : Préparation de complexes progestérone / hydroxypropyl-β-cyclodextrines (HPβCD).

Les complexes progestérone/HPβCD sont préparés en mélangeant 3,615 g de HPβCD avec 3,0 g de progestérone dans 150 ml d'eau sous agitation au moyen d'un agitateur magnétique pendant 24 heures à la température ambiante. Après cela, le mélange est filtré (0,45 µm). La HPβCD et la progestérone sont dosées dans la solution filtrée avant d'être utilisées pour la préparation de nanoparticules chargées de progestérone.

### EXEMPLE 4 : Préparation de nanoparticules de poly(cyanoacrylate d'isobutyle)/HPβCD (PIBCA/ HPβCD) chargées de progestérone.

La solution du complexe progestérone/HPβCD obtenu comme décrit dans l'exemple 3 est diluée pour obtenir des concentrations de 2,5, 5,0 7,5, 10,0, 12,5, 15,0 et 20,0 mg/ml de HPβCD dans le milieu de polymérisation. Les nanoparticules sont préparées comme dans l'exemple 1, en absence ou en présence de 1% (p/v) de poloxamer 188.

### EXEMPLE 5 (témoin) : Préparation de nanoparticules de poly(cyanoacrylate d'isobutyle) chargées de progestérone et dépourvues de HPβCD.

Des nanoparticules de poly(cyanoacrylate d'isobutyle) sont préparées en l'absence de cyclodextrine dans le milieu de polymérisation, pour servir de témoin. Les nanoparticules chargées de progestérone sont préparées en dissolvant le principe actif dans de l'acide chlorhydrique dilué (pH 2,0) en présence de 1% (p/v) de poloxamer 188 (environ 60 µg/ml, correspondant à la solubilité maximale dans ce milieu). Le processus de polymérisation est mis en oeuvre comme décrit à l'exemple 1.

### EXEMPLE 6 : Dosage de la progestérone et de la HPβCD dans les nanoparticules obtenues.

Les différentes suspensions de nanoparticules sont centrifugées à 82 000 g pendant 30 à 40 min à 25°C (Beckman, L5-65 Ultracentrifuge, rotor de type 70,1 Ti) et remises en suspension dans 5 ml d'eau distillée. Les suspensions sont finalement lyophilisées (Christ HED Freeze Drier, Allemagne).
Pour doser la charge de progestérone dans les nanoparticules, les produits lyophilisés sont dilués dans de l'acétonitrile de qualité HPLC et les solutions sont analysées par chromatographie liquide haute performance (HPLC). Le système de HPLC consiste en une unité de délivrance de solvant 510 de Waters (Saint-Quentin-en-Yvelines, France), un préleveur automatique d'échantillons WISP 712, une colonne (250 x 4,6 mm) Nova-Pak C18 4 µm, un détecteur d'absorbance 486 qui fonctionne à 245 nm et est en interface avec un module de données 746. Le débit est de 1,0 ml/min et la phase mobile est constituée d'eau et d'acétonitrile (40:60) où la durée de rétention est d'environ 12 min. Les résultats sont exprimés en tant que moyenne de trois dosages.
Pour quantifier la HPβCD, les nanoparticules lyophilisées sont hydrolysées au moyen de NaOH 0,2 M pendant 12 heures, le pH est ajusté à 7,0 (±0,5) et la HPβCD est quantifiée par dosage spectrophotométrique de la décoloration de solutions de phénolphtaléïne en présence de HPβCD. En effet, la phénolphtaléïne forme des complexes d'inclusion stables et incolores avec les cyclodextrines (CD) (5). Par conséquent, l'intensité de la couleur d'une solution de phénolphtaléine dans du tampon au borate alcalin décroît proportionnellement à la quantité de CD en solution.
Des solutions de référence sont préparées en diluant des solutions-mères de CD dans de la solution tampon de borate alcalin à pH 10,0 contenant 2% d'une solution éthanolique de phénolphtaléïne 0,006 M. Les courbes de référence (λ = 550 nm) sont linéaires pour les concentrations en CD allant de 1 à 100 µg/ml. Les échantillons sont additionnés de 4 parties de solution tampon contenant de la phénolphtaléïne et testées directement.

### EXEMPLE 7 : Caractérisation des nanoparticules.

La distribution granulométrique, la taille moyenne et la polydispersité des nanoparticules sont estimées par diffusion de la lumière laser en utilisant un NS Coulter Nanosizer (Coultronics, Margency, France). Les échantillons sont dispersés dans de l'eau MilliQ (résistivité > 18 MΩ, Millipore, Saint-Quentin-en-Yvelines, France). Chaque analyse dure 200 s. La température est de 20°C et l'angle d'analyse est de 90°. Le potentiel zéta des particules en suspension dans l'eau MilliQ est déterminé par vélocimétrie Doppler au laser (Zetasizer 4, Malvern, Angleterre).

### RÉSULTATS DES EXEMPLES 1 À 7.

Les caractéristiques des particules préparées en présence de 5 mg/ml de différentes cyclodextrines et 1% de poloxamer 188 (moyenne de 3 préparations répétées ± SEM) sont rassemblés dans le tableau I ci-dessous.

**Tableau I**

| CD (5 mg/ml) | Taille (nm) ± S.D. | Potentiel ζ (mV) ± S.D. | Teneur en CD (µg de CD/mg de nanoparticules) |
|---|---|---|---|
| alpha | 228 ± 69 | -34,4 ± 4,0 | ND |
| béta | 369 ± 7 | -24,7 ± 8,2 | 360 |
| gamma | 286 ± 9 | -22,9 ± 0,6 | 240 |
| HPalpha | 244 ± 25 | -27,0 ± 2,2 | ND |
| HPbéta | 103 ± 6 | -8,6 ± 0,9 | 247 |
| HPgamma | 87 ± 3 | -2,6 ± 2,2 | 220 |
| SBEbéta | 319 ± 10 | -45,4 ± 2,4 | ND |
| CD = cyclodextrine | | | |
| HP = hydroxypropyl | | | |
| SBE = éther de sulfobutyle | | | |

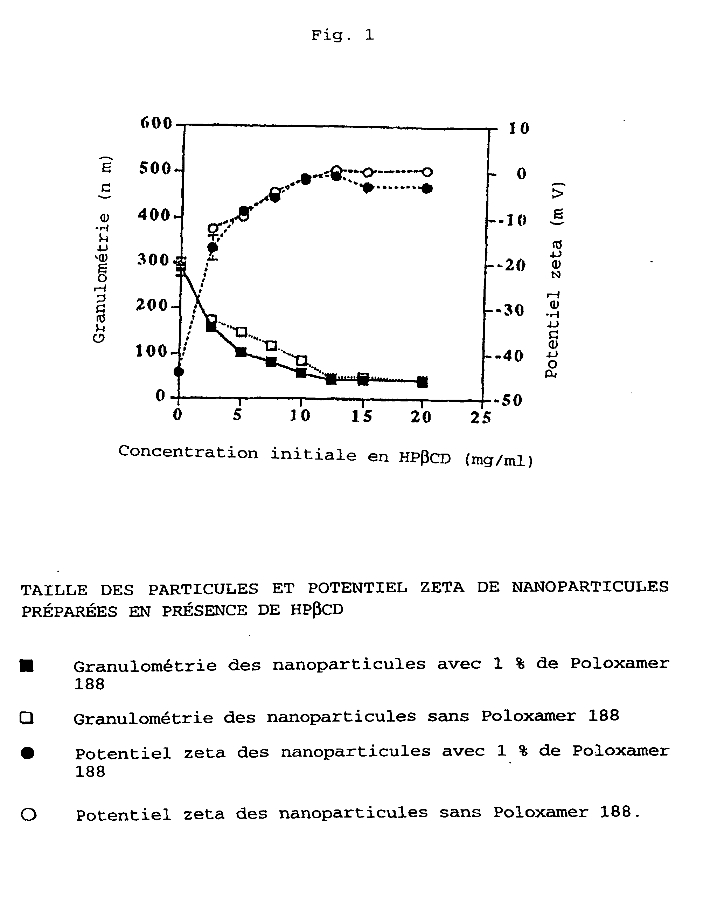

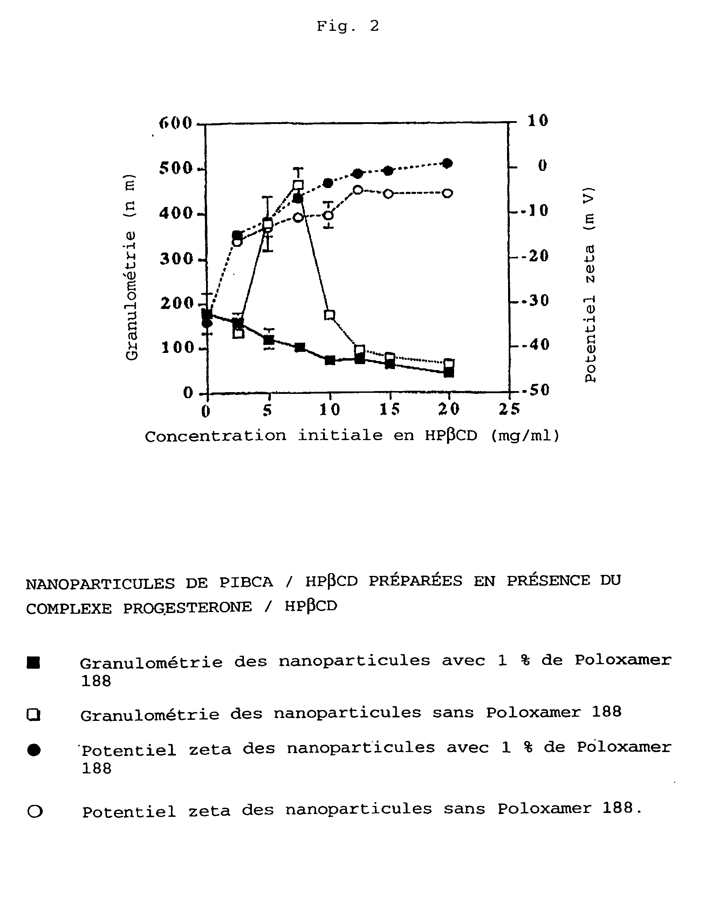

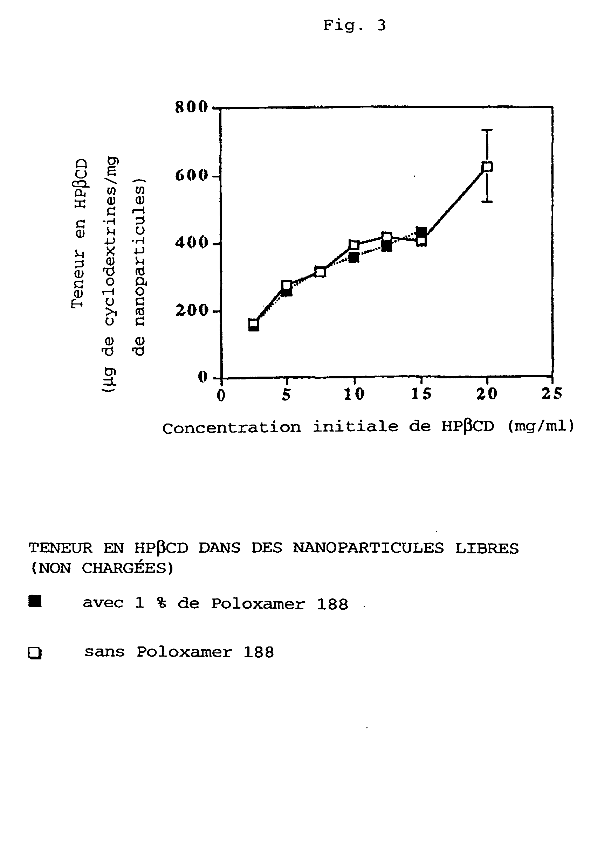

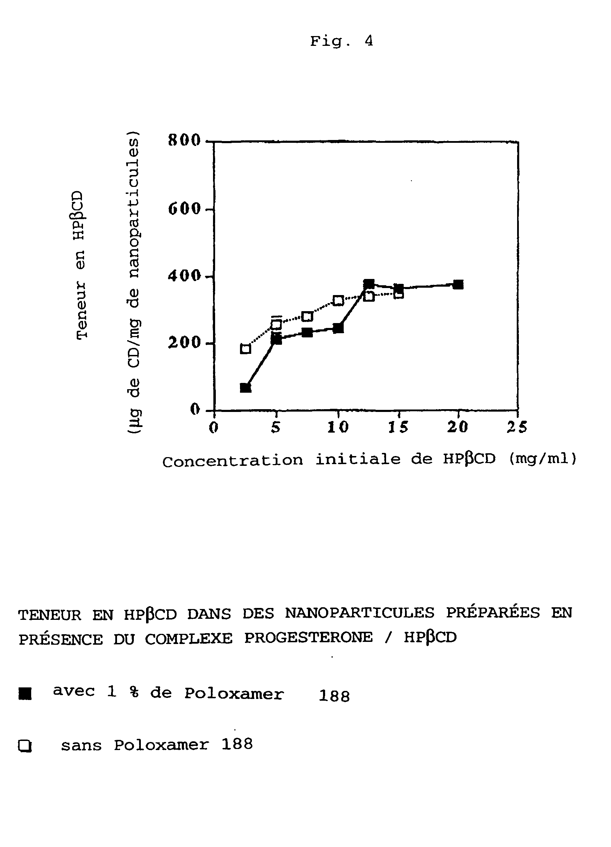

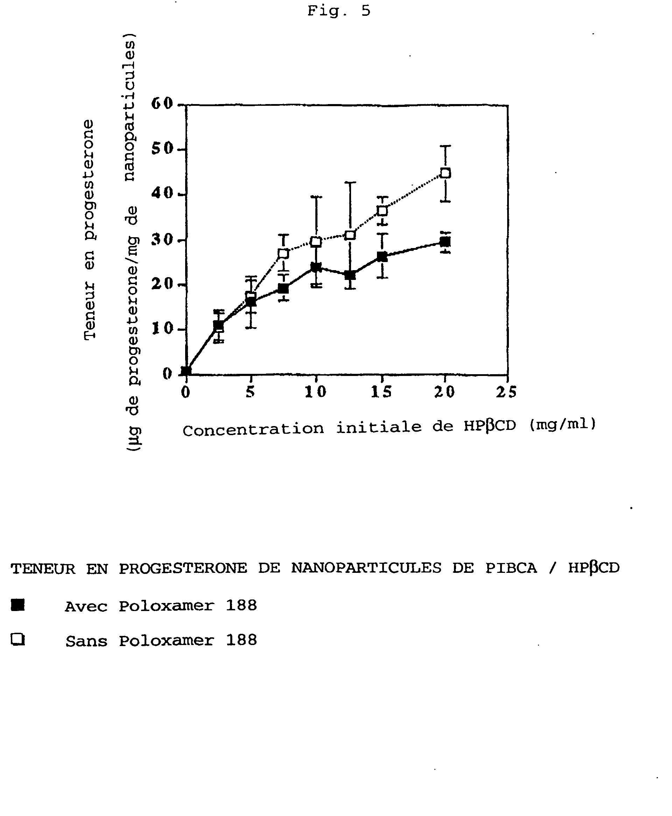

La taille des particules, le potentiel zéta, la teneur en cyclodextrine et la stabilité (valeurs non représentées) sont influencés par la nature de la cyclodextrine.
La quantité des différentes cyclodextrines liées aux particules est dans la gamme de 20 à 35% (p/p) du poids total des particules.
Les nanoparticules formulées avec la HPβCD sont les plus intéressantes car elles présentent une granulométrie moyenne inférieure à 100 nm et un potentiel zéta voisin de zéro mV. En outre, l'HPβCD présente une très grande solubilité dans le milieu de polymérisation et une excellente tolérabilité. Elle permet par ailleurs l'encapsulation de nombreuses substances. Par conséquent, les études complémentaires ont été effectuées avec la HPβCD.
En présence de HPβCD dans le milieu de polymérisation, l'addition de l'agent tensio-actif poloxamer 188 n'est pas essentielle pour la production de nanoparticules.
D'une part, comme le montre la Figure 1, la taille et le potentiel zéta des particules ne sont pas modifiés par la présence de poloxamer 188.
D'autre part, la concentration de HPβCD influence considérablement la taille et le potentiel zéta. Une augmentation de la concentration de HPβCD de 0 à 12,5 mg/ml conduit à une diminution de la taille des particules de 300 nm à moins de 50 nm. De même, le potentiel zéta des particules décroît progressivement de valeurs fortement négatives (-40 mV) à un potentiel de surface proche de 0 mV. Ces tendances sont généralement maintenues lorsque les nanoparticules sont préparées en présence de progestérone, comme le montre la Figure 2. Par rapport aux particules dépourvues de progestérone, le potentiel zéta est légèrement négatif dans la gamme de concentration de HPβCD à l'étude. De plus, en l'absence de poloxamer 188, on observe une augmentation rapide de la taille des nanoparticules jusqu'à 450 nm, suivie d'une diminution rapide lorsque la concentration de HPβCD est supérieure à 10 mg/ml. Ce phénomène est supprimé en présence de poloxamer 188. L'addition de HPβCD dans le milieu de polymérisation conduit à l'association de grandes quantités de HPβCD aux nanoparticules, comme le montre la Figure 3. La quantité de HPβCD associée aux particules augmente continuellement et peut atteindre 60% du poids des particules. Lorsque les masses initiales de HPβCD et de cyanoacrylate d'isobutyle dans le milieu de polymérisation sont égales, la quantité de HPβCD associée aux particules est d'environ 35%. De plus, l'association de HPβCD aux particules n'est pas influencée par la présence de poloxamer 188. La teneur en HPβCD des nanoparticules n'est pas considérablement affectée par la présence de progestérone dans le milieu de polymérisation, comme le montre la Figure 4. La charge de progestérone des particules augmente de façon spectaculaire lorsque les particules sont préparées en présence de HPβCD. La charge de progestérone, en l'absence de HPβCD est de 0,79 µg/mg de particules et elle augmente progressivement jusqu'à être multipliée par 50, ce qui correspond à 45 µg/mg de particules, comme le montre la Figure 5. Il n'y a pas de différences significatives entre les particules préparées avec ou sans poloxamer 188.
Les caractéristiques des nanoparticules utilisées dans les exemples 8 à 10 qui suivent sont décrites dans le tableau II ci-dessous.

**Tableau II**

| Formulation^{a} | Taille (nm) ± SD | Teneur en HPβCD^{b} | Teneur en principe actif^{c} |
|---|---|---|---|
| 2,5 | 158 ± 22 | 64 ± 5,4 | 10,9 ± 3,3 |
| 10,0 | 70 ± 5 | 240 ± 7,6 | 23,9 ± 4,4 |
| a = concentration initiale en HPβCD dans le milieu de polymérisation (mg/ml) | | | |
| b = µg de HPβCD par mg de nanoparticules | | | |
| c = µg de progestérone par mg de nanoparticules | | | |

### EXEMPLE 8 : Libération de la progestérone in vitro à partir de nanoparticules de PIBCA/HPβCD.

Une quantité pesée de nanoparticules lyophilisées (contenant 1% (p/v) de glucose)est mise dans un flacon contenant 15 ml d'une solution tampon au borate alcalin (ABB) (pH 8,4) ou d'ABB contenant des estérases (25 et 100 UI) ou d'ABB/poly(éthylèneglycol) 400 (PEG) à 20 et 40% (v/v). Les échantillons sont agités au moyen d'un agitateur magnétique à 200 tr/min et 37°C, et prélevés à des intervalles prédéterminés. Les suspensions sont centrifugées à 82 000 g pendant 30 minutes à 20°C puis la teneur en progestérone du surnageant est dosée pour tous les milieux et la teneur en HPβCD pour les milieux au PEG. La teneur en progestérone est dosée par HPLC comme décrit plus haut, avec injection de 100 µl pour les échantillons incubés dans des milieux à l'ABB et 20 µl pour les milieux au PEG.
Tous les essais sont réalisés dans des conditions telles que la concentration en principe actif dans la phase de libération soit maintenue au-dessous de 10% de saturation.

### EXEMPLE 9 : Libération de la HPβCD in vitro à partir de nanoparticules de PIBCA/HPβCD.

L'étude de la libération de la HPβCD est effectuée comme celle de la progestérone dans du milieu ABB avec quantification de la teneur en CD après ultracentrifugation, par complexation avec de la phénolphtaléine comme décrit plus haut. La concentration de CD à 100% de libération est d'environ 100 µg/ml.

### EXEMPLE 10 : Calorimétrie par analyse différentielle (DSC).

Les études de DSC sont effectuées en utilisant un calorimètre d'analyse différentielle Perkin Elmer DSC-7. La température est calibrée en utilisant le point de transition de fusion de l'indium. Des échantillons pesant environ 4 mg sont placés dans des capsules d'aluminium et chauffés de 0 à 250°C à une vitesse d'exploration de 10°C/min.

### RÉSULTATS DES EXEMPLES 8 À 10.

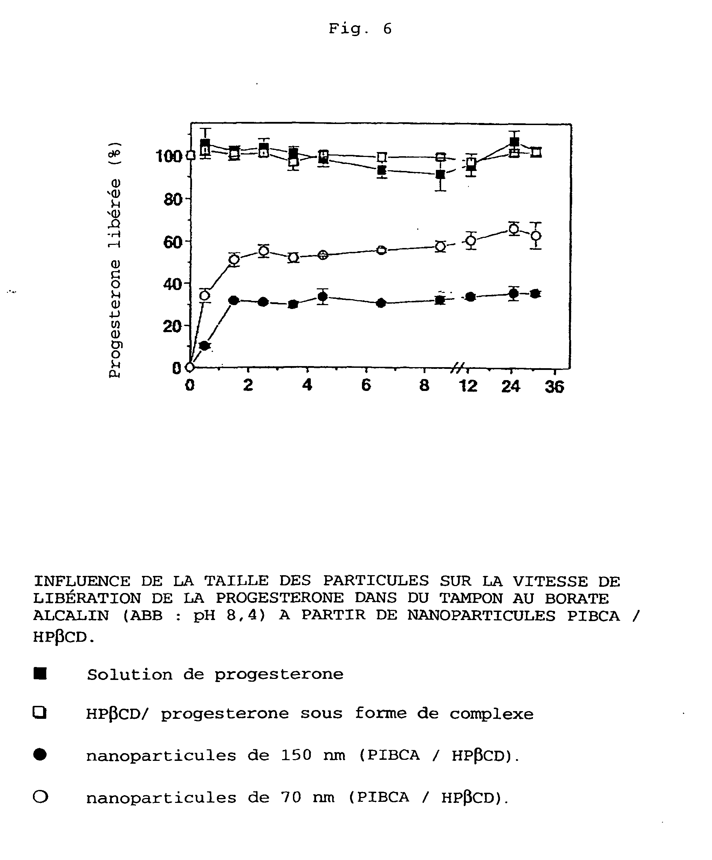

La Figure 6 des dessins annexés représente le profil de libération de la progestérone à partir de nanoparticules combinées de PIBCA/HPβCD dans l'ABB (pH 8,4). Sur ce graphique on peut observer une courbe de libération biphasique avec une libération initiale rapide (effet d'éclatement) dans la première heure pour les deux formulations testées (environ 10 et 34% des nanoparticules de 150 et 70 nm, respectivement). Cette libération rapide pourrait être attribuée à la fraction de progestérone qui est adsorbée ou faiblement liée à la grande surface générée par la formation de nanoparticules plutôt qu'au complexe progestérone/CD incorporé dans le réseau polymère. La seconde phase correspond à une libération exponentielle plus lente avec environ 35 et 62% de progestérone libérée à partir de nanoparticules de 150 et 70 nm, respectivement. La phase de libération ralentie peut être le résultat d'une simple diffusion vers l'extérieur de la progestérone à partir des nanoparticules ou de la pénétration de la solution de libération dans les nanoparticules avec dissolution de la progestérone, suivie de sa diffusion à l'extérieur.
Les études *in vitro* démontrent que différents facteurs peuvent affecter la libération de principes actifs à partir de systèmes colloïdaux. Ces facteurs comprennent la taille et la morphologie des particules, la charge en principe actif et la solubilité de celui-ci (6, 7, 8). Conforménent à ce qui a été observé dans les travaux antérieurs, les nanoparticules les plus petites (70 nm) avec une charge supérieure en principe actif (24 µg/mg) présentent une libération plus rapide que les particules plus grandes (170 nm) avec une charge plus faible en progestérone (10,5 µg/mg). La taille moyenne et la charge de principe actif des nanoparticules représentent les facteurs majeurs de la vitesse de libération, avec une réduction de la phase rapide pour les nanoparticules plus grandes.

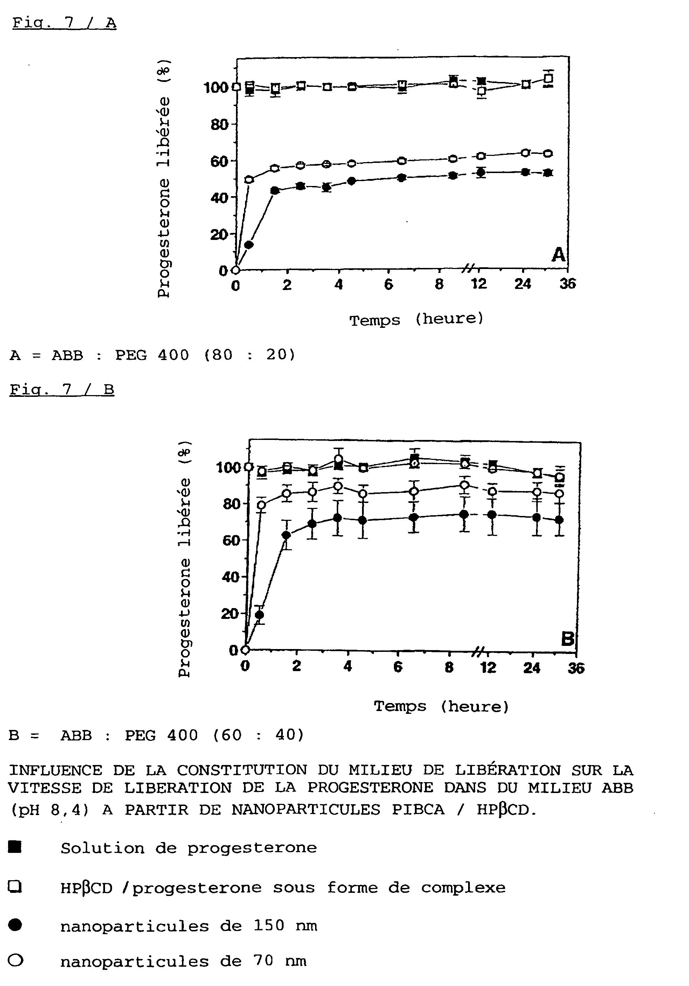

La Figure 7 montre les profils de libération de la progestérone à partir de nanoparticules de PIBCA/HPβCD en présence de PEG 400 (20 et 40%) en tant qu'agent solubilisant. L'utilisation de ce type de milieu permet de réduire le volume de milieu de libération et par conséquent la concentration en principe actif permettant une meilleure détection (9). Dans un cas comme celui-ci où des solvants ou des agents solubilisants non aqueux sont employés, on peut obtenir des informations concernant le mécanisme de libération. Comme montré à la Figure 7, le profil de libération n'est pas identique pour les deux milieux, ce qui signifie que la libération est fortement influencée par la concentration en PEG. Par conséquent, la libération de la progestérone doit être déterminée par la pénétration du solvant dans la matrice polymère, avec dissolution et diffusion à l'extérieur du principe actif à partir des nanoparticules. Au contraire, quand la libération du principe actif résulte de la simple diffusion à travers une matrice polymère, la composition du solvant de libération ne peut pas influencer la libération de principe actif (10).

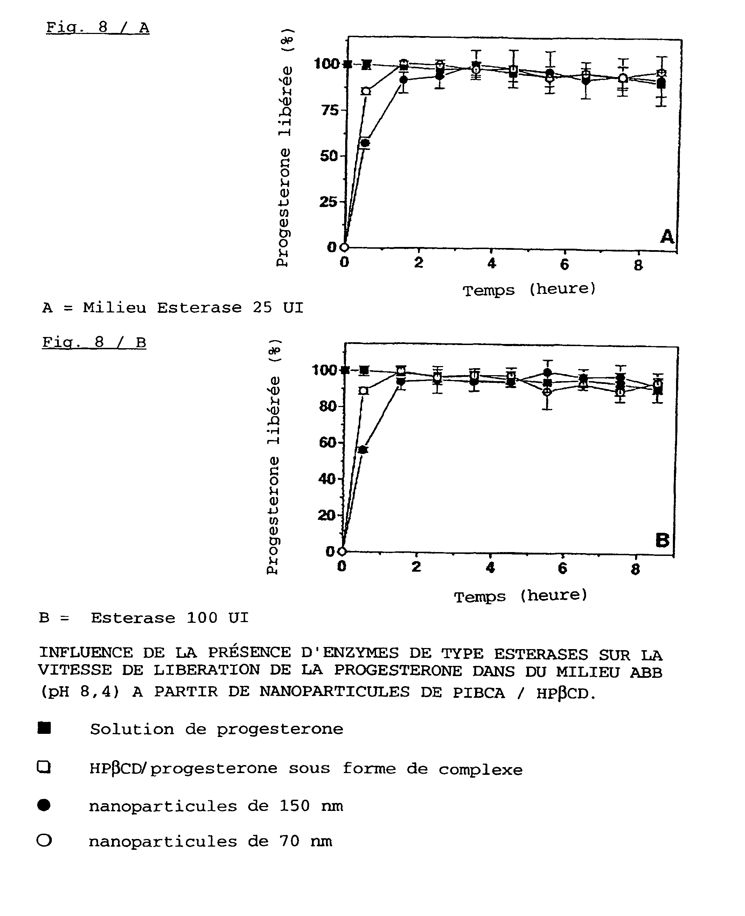

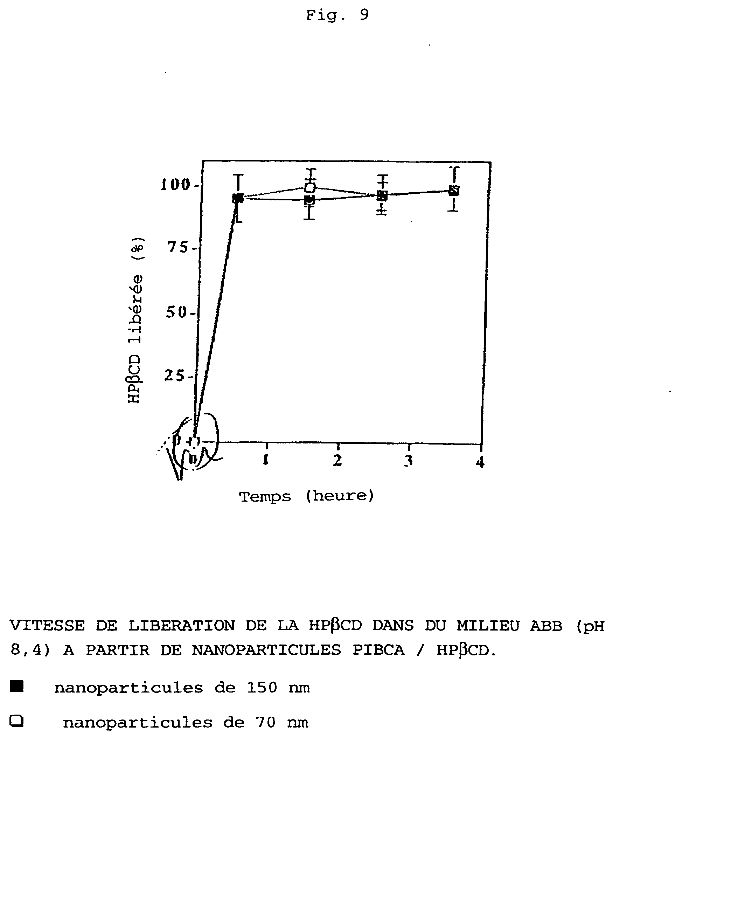

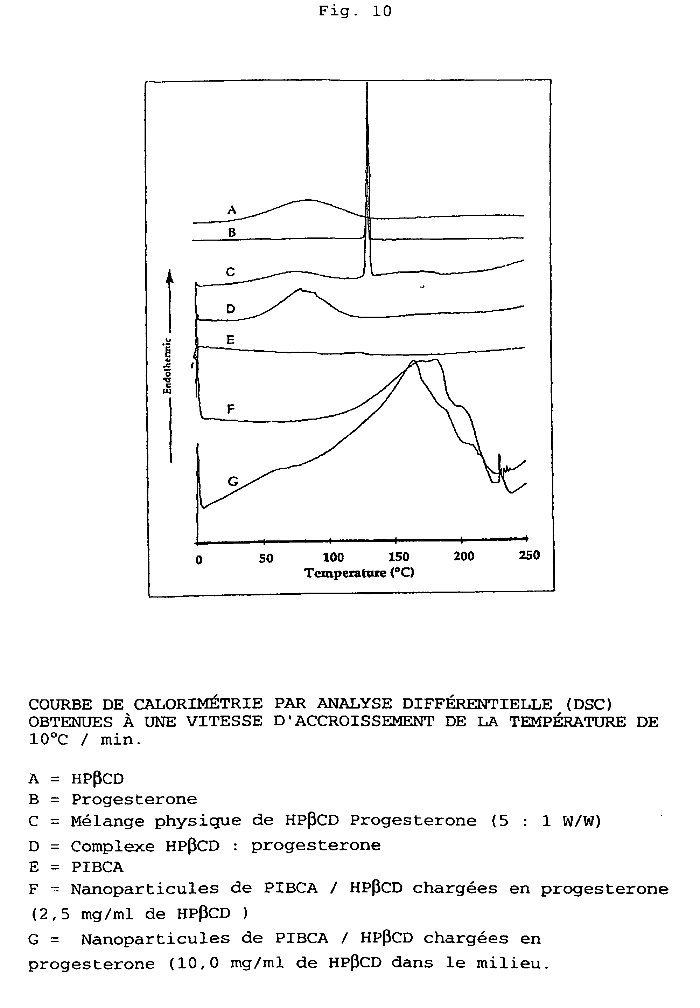

Le procédé de préparation de nanoparticules qui consiste à ajouter le monomère à une solution aqueuse d'agent tensio-actif et à agiter pour obtenir des micelles (2), peut déterminer la distribution du principe actif dans les micelles pendant l'étape de polymérisation.
La libération rapide observée dans les figures 6 et 7 suggère que la surface des particules a été enrichie en progestérone au cours de l'étape de polymérisation. Par ailleurs, une forte proportion du principe actif a pu être piégée dans le réseau de polymère qui pouvait avoir une structure interne hautement poreuse (11). Ce fait pourrait expliquer l'augmentation de la vitesse de libération lorsque la concentration en PEG augmente (Figure 7), le PEG pénétrant dans la structure à des vitesses différentes selon la constitution du milieu de libération puis modifiant la diffusion du principe actif vers l'extérieur.
Malgré une augmentation très importante de la vitesse de libération obtenue grâce à l'addition de PEG dans le milieu de libération, la libération de la progestérone n'atteind pas 100% (elle est d'environ 75 et 82%, respectivement avec 40% de PEG).
Au contraire, la présence d'enzymes de type estérase dans le milieu de libération conduit à une libération plus rapide que dans une solution de libération dépourvue d'estérase et la quantité de progestérone libérée est très proche de 100% pour les deux formulations testées et pour les deux concentrations d'enzyme (Figure 8). Ces faits peuvent suggérer que les molécules de progestérone sont, au moins en partie, piégée à l'état moléculaire dans la matrice polymère de la nanoparticule de l'invention et/ou liées au réseau de cyanoacrylate d'isobutyle (12). L'utilisation d'enzymes de type estérase dans le milieu de libération conduit à une dégradation ou une dissolution des chaînes polymères des nanoparticules de poly(cyanoacrylate). Dans ce cas, les principes actifs immobilisés dans la matrice sont alors libérés par la dégradation progressive de celle-ci.
La bioérosion provoquée par l'hydrolyse de la liaison ester des chaînes latérales du PIBCA est le mécanisme qui permet une accélération significative de la libération de progestérone, ce qui correspond aux résultats rapportés par d'autres auteurs (12, 13). Parfois, les études de libération de principes actifs effectuées dans des milieux contenant des estérases ne conduisent pas à une libération de 100% du principe actif incorporé (12, 14, 15). Il est suggéré qu'alors il existe la possibilité d'une liaison entre les chaînes de PIBCA et les molécules de principe actif (12, 14). Les profils de libération de la cyclodextrine à partir des nanoparticules représentés à la Figure 9 montrent une libération très rapide et très proche de 100% dans la première heure, ce qui montre que ces molécules ne sont pas liées de manière chimique au polymère mais vraisemblablement simplement adsorbées ou piégées dans le polymère.
Les profils de DSC des échantillons contenant de la HPβCD montrent une transition endothermique large, reproductible dans la gamme de 30 à 90°C avec des températures de début comprises dans cette gamme (Figure 10 a, c et d). Ce pic asymétrique a été attribué à l'élimination d'eau. Les échantillons contenant de la progestérone (mélange physique et progestérone seule) présentent un pic endothermique prononcé à environ 130°C, ce qui correspond à la température de transition de fusion de la progestérone sous forme cristalline (Figure 10 b et c). Le complexe HPβCD : progestérone ne présente que la transition endothermique dans la gamme de 30 à 90°C décrite ci-dessus, avec disparition de la transition de fusion de la forme cristalline de la progestérone (Figure 10 d), ce qui suggère que le principe actif est dispersé à l'état moléculaire dans la cavité des molécules de cyclodextrine. Sous la même forme, des échantillons de nanoparticules de PIBCA/HPβCD chargées de progestérone ne présentent pas de pic endothermique prononcé qui, dans ce cas, est remplacé par une transition endothermique large dans la gamme de 130 à 170°C (Figure 10 e et f). Ce phénomène suggère que la progestérone se trouve à l'état moléculaire soit dissoute dans le polymère soit incluse dans les cyclodextrines associées au nanoparticules selon l'invention. Sous cette forme, l'ensemble des résultats concernant la libération de la cyclodextrine et de la progestérone dans les différents milieux et les courbes de DSC, ajoutés aux données de la littérature, indiquent que la morphologie des nanoparticules pourrait être représentée par un noyau polymère contenant une fraction du principe actif à l'état moléculaire, avec une surface enrichie par des complexes cyclodextrine:progestérone. Cette structure pourrait expliquer la libération biphasique de la progestérone avec une première phase rapide due peut-être à la désorption du complexe cyclodextrine:progestérone à partir de la surface, et une seconde phase très lente comprenant la diffusion de la progestérone vers l'extérieur, à travers le réseau polymère.

### EXEMPLE 11 : Préparation de nanoparticules de poly (cyanoacrylate d'isobutyle) /HPβCD chargées en divers principes actifs.

Des complexes de prednisolone, de spironolactone, de testotérone, de progestérone, de danazol et d'acetate de megestrol ont été obtenues en mélangeant 300 mg HPβCD avec 15 mg de stéroides dans 15 ml d'eau à 37°C pendant 72 heures sous agitation magnétique. Les suspensions ont été filtrées (0.45 mm) et les concentrations en cyclodextrine et en principe actif ont été dosées selon l'exemple 12 ci-après. Des nano particules de poly(cyanoacrylate d'isobutyle)/HPβCD sont préparées comme dans l'exemple 1 en ajoutant une solution des complexes formés contenant 10mg/ml de HPβCD dans une solution de poloxamer à 1% p/V.

### EXEMPLE 12 (témoin) : Préparation de nanoparticules de poly (cyanoacrylate d'isobutyle) chargées en divers principes actifs.

Des solutions d'hydrocortisone, de prednisolone, de spironolactone, de testoterone, de progestérone, de danazol et d'acetate de megestrol à des concentrations correspondant à la concentration à saturation dans du poloxamer 188 (1% p/v) ont été ajoutées séparemment dans les milieux de polymérisation. Des nanoparticules de poly(cyanoacrylate d'isobutyle) chargées en divers principes actifs ont alors été préparées selon l'exemple 1, mais en l'absence de HPβCD.

### EXEMPLE 13 : Dosage de l'hydrocortisone, de la prednisolone, de la spironolactone, de la testoterone, de la progestérone, du danazol, de l'acetate de megestrol et de HPβCD.

Les différents stéroides ont été dosés selon l'exemple 6 qui permet le dosage de ces différentes substances dans les mêmes conditions analytiques.

L'HPβCD a été dosée également selon l'exemple 6.

### EXEMPLE 14 : Caractéristiques de taille et de potentiel zéta des nanoparticules préparées selon l'invention en présence ou en absence de poloxamer 188

Les nanoparticules préparées selon l'exemple 11 et l'exemple 12 ont été caractérisées selon l'exemple 7. La taille des particules chargées en stéroides était généralement diminuée et était proche de 100 nm environ lorsque les nanoparticules selon l'invention étaient préparées en l'absence de poloxamer 188 et seulement en présence de l'HPβCD suggérant un masquage des charges par les molécules de cyclodextrine localisées à la surface des particules.

Le tableau III ci-dessous rapporte le chargement de la drogue par des nanoparticules de poly(cyanoacrylate d'alkyle) ou des nanoparticules de poly(cyanoacrylate d'alkyle) et hydroxypropyl-β-cyclodextrine et le contenu en cyclodextrine correspondant (moyenne de 3 valeurs).

**Tableau III**

| Échantillon | | CD (mg/g) | chargement de la drogue (mg/g) |
|---|---|---|---|
| PIBCA | | | |
| PIBCA | hydrocortisone (HD) | - | 2,19 |
| | PE | - | 0,12 |
| | sprironolactone (SP) | - | 7,65 |
| | testosterone (TE) | - | 2,27 |
| | megestrol acetate (AM) | - | 0,25 |
| | danazol (DA) | - | 0,34 |
| | progesterone (PO) | - | 0,79 |
| PIBCA/HPβCD | HD | 180 | 15,3 |
| | PE | 210 | 15,5 |
| | SP | 230 | 53,0 |
| | TE | 180 | 19,5 |
| | AM | 220 | 1,4 |
| | DA | 280 | 11,2 |
| | PO | | 24,0 |

### RÉSULTATS DES EXEMPLES 11 À 14 : Augmentation de la charge en stéroides de nanoparticules selon l'invention.

Les valeurs des chargements en stéroides exprimés en valeur absolues pour les nanoparticules selon l'invention ou les particules témoins sont rassemblées dans le tableau IV (moyenne de trois préparations). Le calcul des valeurs d'accroissement de la charge des particules montre que l'augmentation de la charge peut atteindre 129 fois, dans le cas de la prednisolone.

**Tableau IV**

| Stéroides | Chargement des nanoparticules de PIBCA sans HPβCD (mmole/g) | Chargement des nanoparticules combinées de PIBCA et HPβCD (mmole / g) | Augmentation du chargement (nombre de fois) |
|---|---|---|---|
| Hydrocortisone | 6,04 | 42,21 | 7,0 |
| Prednisolone | 0,33 | 43,00 | 129,2 |
| Spironolactone | 18,36 | 127,23 | 6,9 |
| Testoterone | 7,87 | 67,6 | 8,6 |
| Acetate de megestrol | 0,65 | 3,64 | 5,6 |
| Danazol | 1,01 | 33,19 | 32,9 |
| Progesterone | 2,51 | 69,60 | 27,7 |

### RÉFÉRENCES

1) J. Kreuter, Colloidal Drug Delivery Systems, Marcel Decker, New York, 1994, 219-342.
2) Brevets EP-B-0 007 895 (US-A-4,329,332 & US-A-4,489,055) et EP-B-0 064 967 (US-A-4,913,908).
3) C. Cuvier et coll., Biochem. Pharmacol., 44, 509-517 (1992).
4) AC de Verdière et coll., British Journal of Cancer 76(2), 198-205 (1997).
5) M. Vikmon, Proceed. First International Symposium on Cyclodextrins, Budapest, 1981, 69-74.
6) E. Allémann et coll., Pharm. Res., 10, 1732-1737 (1993).
7) J.-C. Leroux et coll., J. Control. Rel., 39, 339-350 (1996).
8) N. Erden et coll., International Journal of Pharmaceutics, 137, 57-66 (1996).
9) J.-P.Benoit et coll., Microspheres and Drug Therapy. Pharmaceutical, Immunological and Medical Aspects, Elsevier, Amsterdam, 1984, 91-102.
10) C. Washington, Int. J. Pharm., 58, 1-12 (1990).
11) P. Couvreur et coll., J. Pharm. Sci., 68, 1521-1524 (1979).
12) F. Fawaz et coll., International Journal of Pharmaceutics, 154, 191-203 (1997).
13) J. L. Grangier et coll., J. Control. Rel., 15, 3-13 (1991).
14) Ch. Tasset et coll., J. Control. Rel., 33, 23-30 (1995).
15) B. Seijo et coll., Int. J. Pharm., 62, 1-7 (1990).

## Revendications

1. (non modifiée) Nanoparticules **caractérisées en ce qu'**elles comprennent au moins un polymère, au moins un principe actif et au moins un oligosaccharide cyclique.

2. (non modifiée) Nanoparticules selon la revendication 1, **caractérisées en ce que** l'un au moins des polymères est un poly(cyanoacrylate d'alkyle) dans lequel le groupe alkyle, linéaire ou ramifié, comprend de 1 à 12 atomes de carbones.

3. (non modifiée) Nanoparticules selon l'une des revendications 1 ou 2, **caractérisées en ce que** l'oligosaccharide cyclique est une cyclodextrine neutre ou chargée, native, branchée ou polymérisée ou modifiées chimiquement.

4. (non modifiée) Nanoparticules selon l'une quelconques des revendications précédentes, **caractérisées en ce que** l'oligosaccharide cyclique est une cyclodextrine modifiée chimiquement par substitution d'un ou plusieurs hydroxypropyles par des groupements alkyle, aryle, arylalkyle, glycosidique, ou par etherification, estérification avec des alcools ou des acides aliphatiques.

5. (non modifiée) Nanoparticules selon l'une quelconques des revendications précédentes, **caractérisées en ce qu'**elles présentent une taille comprise entre 300 et moins de 50 nm.

6. (non modifiée) Nanoparticules selon l'une quelconque des revendications précédentes, **caractérisées en ce que** le principe actif est hydrophile, hydrophobe, amphiphile et/ou insoluble.

7. (non modifiée) Nanoparticules selon l'une quelconque des revendications précédentes, **caractérisées en ce que** le principe actif est choisi parmi les anticancéreux, les molécules antisens, les antiviraux, les antibiotiques, les protéines, polypeptides, polynucléotides, les substances vaccinantes, les immunomodulateurs, les stéroïdes, les analgésiques, les antimorphiniques, les antifongiques et antiparasitaires.

8. (non modifiée) Nanoparticules selon la revendication 7, **caractérisées en ce que** le principe actif est le taxol ou l'un de ses dérivés.

9. (non modifiée) Nanoparticules selon la revendication 7, **caractérisées en ce que** le principe actif est la doxorubicine ou l'un de ses dérivés.

10. (non modifiée) Nanoparticules selon la revendication 7, **caractérisées en ce que** le principe actif est un dérivé du platine.

11. (non modifiée) Nanoparticules selon l'une quelconque des revendications précédentes, **caractérisées en ce que** le principe actif est présent en une quantité de 0,01 à 300 mg/g de nanoparticules.

12. (non modifiée) Nanoparticules selon l'une quelconque des revendications précédentes, **caractérisées en ce que** la proportion d'oligosaccharide cyclique est de 0,1 à 70 % en poids.

13. (non modifiée) Procédé de préparation de nanoparticules à base d'un polymère selon l'une quelconque des revendications 1 à 12, **caractérisé en ce qu'**il comprend les étapes consistant à :
a) préparer un complexe d'au moins un principe actif avec au moins un oligosaccharide cyclique, en solution dans un solvant aqueux ou non aqueux,
b) ajouter progressivement au moins un monomère du polymère dans la solution obtenue à l'étape (a), et
c) effectuer une polymérisation, par exemple anionique ou inductible par d'autres agents notamment photochimiques, de ce monomère éventuellement en présence d'un ou plusieurs agents tensio-actif et/ou stabilisant.

14. (non modifiée) Procédé de préparation de nanoparticules à base d'un polymère selon l'une quelconque des revendications 1 à 12, **caractérisé en ce qu'**il comprend les étapes consistant à :
- préparer des nanoparticules à base d'un polymère et plus particulièrement des poly(cyanoacrylate d'alkyle) et d'un oligosaccharide cyclique,
- associer auxdites nanoparticules le principe actif.

15. (non modifiée) Procédé de préparation de nanoparticules à base d'un polymère selon la revendication 14, **caractérisé en ce qu'**il comprend les étapes consistant à:
a) préparer une solution d'au moins un oligosaccharide cyclique dans un solvant aqueux ou non aqueux,
b) ajouter progressivement au moins un monomère du polymère, et plus particulièrement le cyanoacrylate d'alkyle monomère dans la solution de l'étape (a), et
c) effectuer une polymérisation de préférence anionique mais également inductible par d'autres agents notamment photochimiques de ce monomère, éventuellement en présence d'un ou plusieurs agents tensio-actif et/ou stabilisant,
d) après un contrôle et une purification éventuelle des nanoparticules obtenus à l'étape (c), incuber lesdites particules dans une solution du principe actif dans un solvant aqueux ou non aqueux.

16. (non modifiée) Procédé de préparation de nanoparticules à base de poly(cyanoacrylate d'alkyle) selon l'une des revendications 13 à 15, **caractérisé en ce qu'**à l'étape (b) on ajoute progressivement au moins un cyanoacrylate d'alkyle monomère.

17. (non modifiée) Procédé selon l'une quelconque des revendications 13 à 16, **caractérisé en ce que** aux étapes (a), (b) et (d), le solvant est avantageusement choisi de façon à ce que, tout en maintenant des conditions favorables à la polymérisation des polymères et plus particulièrement des poly(cyanoacrylate d'alkyle), la solubilité du principe actif et de l'oligosaccharide cyclique soit maximale dans le milieu défini par ce solvant.

18. (non modifiée) Procédé selon l'une quelconque des revendications 13 à 17, **caractérisé en ce que** l'étape (c) est effectuée sans agent tensio-actif et/ou stabilisant.

19. (non modifiée) Procédé selon l'une quelconque des revendications 13 à 17, **caractérisé en ce que** dans l'étape (a) la proportion d'oligosaccharide cyclique est en général de 0,1 à 70 % en poids par rapport audit principe actif.

20. (non modifiée) Utilisation du procédé selon l'une quelconque des revendications 13 à 19 pour fabriquer un médicament à effet ciblé et à index thérapeutique amélioré.

21. (modifiée) Nanoparticule comprenant l'association d'un polymère et plus particulièrement des poly(cyanoacrylate d'alkyle) et d'un oligosaccharide cyclique, susceptible d'être obtenu par les étapes (a) à (c) d'un procédé selon la revendication 15.

22. (non modifiée) Nanoparticules selon la revendication 21, **caractérisées en ce que** l'oligosaccharide cyclique est une cyclodextrine neutre ou chargée, native, branchée ou polymérisée ou modifiées chimiquement.

## Patentansprüche

1. Nanopartikel, **dadurch gekennzeichnet, dass** sie mindestens ein Polymer umfassen, mindestens einen Wirkstoff und mindestens ein Oligosaccharid.

2. Nanopartikel gemäß Anspruch 1, **dadurch gekennzeichnet, dass** zumindest eines der Polymere ein Poly- (Alkyl-Cyanoakrylat) ist, in dem die lineare oder verzweigte Alkylgruppe zwischen 1 und 12 Kohlenstoffatome umfasst.

3. Nanopartikel gemäß einem der Ansprüche 1 bis 2, **dadurch gekennzeichnet, dass** das zyklische Oligosaccharid ein neutrales oder geladenes, ein natürlich vorkommendes, verzweigtes oder polymerisiertes oder chemisch verändertes Cyclodextrin ist.

4. Nanopartikel gemäß einem der vorherigen Ansprüche, **dadurch gekennzeichnet, dass** das zyklische Oligosaccharid ein Cyclodextrin ist, das durch Ersatz eines oder mehrerer Hydroxypropyle durch Alkylgruppen, Akryl, Akrylalkyl, Glykosid oder durch Veretherung, Veresterung mit Alkoholen oder mit aliphatischen Säuren chemisch verändert wurde.

5. Nanopartikel gemäß einem der vorherigen Ansprüche, **dadurch gekennzeichnet, dass** sie eine Größe zwischen 300 und unter 50 nm haben.

6. Nanopartikel gemäß einem der vorherigen Ansprüche, **dadurch gekennzeichnet, dass** der Wirkstoff hydrophil, hydrophob, amphiphil und/oder unlöslich ist.

7. Nanopartikel gemäß einem der vorherigen Ansprüche, **dadurch gekennzeichnet, dass** der Wirkstoff aus nicht krebserregenden Wirkstoffen, antisensen Molekülen, antiviralen Stoffen, Antibiotika, Proteinen, Polypeptide, Polynukleotide, immunisierende Stoffe, Immunmodulatoren, Steroide, Analgetika, Antimorphine, Antimykotika und Schädlingsbekämpfungsmittel ausgewählt wird.

8. Nanopartikel gemäß Anspruch 7, **dadurch gekennzeichnet, dass** es sich bei dem Wirkstoff um Taxol oder einen seiner Derivate handelt.

9. Nanopartikel gemäß Anspruch 7, **dadurch gekennzeichnet, dass** es sich bei dem Wirkstoff um Doxorubicin oder einen seiner Derivate handelt.

10. Nanopartikel gemäß Anspruch 7, **dadurch gekennzeichnet, dass** es sich bei dem Wirkstoff um ein Platin-Derivat handelt.

11. Nanopartikel gemäß einem der vorherigen Ansprüche, **dadurch gekennzeichnet, dass** der Wirkstoff in einer Menge zwischen 0,01 und 300 mg/g der Nanopartikel vorliegt.

12. Nanopartikel gemäß einem der vorherigen Ansprüche, **dadurch gekennzeichnet, dass** das Mengenverhältnis des zyklischen Ologosaccharides zwischen 0,1 und 70 Gewicht-% liegt.

13. Verfahren zur Vorbereitung von Nanopartikel auf Basis eines Polymers, gemäß einem der Ansprüche 1 bis 12, **dadurch gekennzeichnet, dass** es folgende Schritte umfasst, die in Folgendem bestehen:
a) Vorbereitung eines Komplexes mit mindestens einem Wirkstoff mit mindestens einem zyklischen Oligosaccharid, gelöst in einem wässrigen oder nicht wässrigen Lösungsmittel,
b) Schrittweises Hinzufügen von mindestens einem Monomer des Polymers zu der in dem Schritt (a) erhaltenen Lösung und
c) Durchführung einer Polymerisation, zum Beispiel anionisch oder induktiv durch andere Wirkstoffe, insbesondere photochemisch, bei diesem Monomer, eventuell im Beisein eines oder mehrerer grenzflächenaktiver und/oder stabilisierender Wirkstoffe.

14. Verfahren zur Vorbereitung von Nanopartikel auf Basis eines Polymers, gemäß einem der Ansprüche 1 bis 12, **dadurch gekennzeichnet, dass** es folgende Schritte umfasst, die in Folgendem bestehen:
- Vorbereitung der Nanopartikel auf Basis eines Polymers und genauer gesagt der Poly- (Alkyl-Cyanoakrylat) und eines zyklischen Oligosaccharides,
- Hinzufügen des Wirkstoffs zu den besagten Nanopartikel

15. Verfahren zur Vorbereitung von Nanopartikel auf Basis eines Polymers, gemäß Anspruch 14, **dadurch gekennzeichnet**, das es folgende Schritte umfasst, die in Folgendem bestehen:
a) Vorbereitung einer Lösung mit mindestens einem zyklischen Oligosaccharid in einer wässrigen oder nicht wässrigen Lösung,
b) Schrittweises Hinzufügen von mindestens einem Polymer und genauer gesagt des Alkyl-Cyanoakrylat-Monomer in die Lösung des Schritts (a) und
c) Durchführung einer Polymerisation, vorzugsweise anionisch aber auch induktiv, durch andere Wirkstoffe, insbesondere photochemische Wirkstoffe dieses Monomers, eventuell im Beisein eines oder mehrerer grenzflächenaktiver und/oder stabilisierender Wirkstoffe,
d) nach einer Kontrolle und einer eventuellen Reinigung der im Schritt (c) erhaltenen Nanopartikel, die besagten Partikel in einer Lösung des Wirkstoffs in einem wässrigen oder nicht wässrigen Lösungsmittel inkubieren.

16. Verfahren zur Vorbereitung von Nanopartikel auf Basis von Poly- (Alkyl-Cyanoakrylat) gemäß einem der Ansprüche 13 bis 15, **dadurch gekennzeichnet, dass** man im Schritt (b) schrittweise mindestens ein Alkyl-Cyanoakrylat-Monomer hinzufügt.

17. Verfahren gemäß einem der Ansprüche 13 bis 16, **dadurch gekennzeichnet, dass** bei den Schritten (a), (b) und (d) das Lösungsmittel vorteilhafterweise so ausgewählt wird, dass bei der Polymerisation der Polymere günstige Bedingungen aufrecht erhalten werden und genauer gesagt Alkyl-Cyanoakrylate, wobei die Lösbarkeit des Wirkstoffs und des zyklischen Oligosaccharides in der durch dieses Lösungsmittel festgelegten Umgebung maximal ist.

18. Verfahren gemäß einem der Ansprüche 13 bis 17, **dadurch gekennzeichnet, dass** der Schritt (c) ohne grenzflächenaktiven und/oder stabilisierenden Wirkstoff durchgeführt wird.

19. Verfahren gemäß einem der Ansprüche 13 bis 17, **dadurch gekennzeichnet, dass** bei dem Schritt (a) das Mengenverhältnis des zyklischen Oligosaccharides im Vergleich zu dem besagten Wirkstoff im allgemeinen zwischen 0,1 und 70 Gewicht-% liegt.

20. Einsatz des Verfahrens gemäß einem der Ansprüche 13 bis 19 zur Herstellung eines Medikamentes mit gezielter Wirkung und mit einem verbesserten therapeutischen Index.

21. Nanopartikel inklusive der Verbindung eines Polymers und genauer gesagt von Poly (-Alkyl-Cyanoakrylaten) und einem zyklischen Oligosaccharid, das man durch die Schritte (a) bis (c) eines Verfahrens gemäß dem Anspruch 15 erhalten kann.

22. Nanopartikel gemäß Anspruch 21, **dadurch gekennzeichnet, dass** das zyklische Oligosaccharid ein neutrales oder geladenes, ein natürlich vorkommendes, verzweigtes oder polymerisiertes oder chemisch verändertes Cyclodextrin ist.

## Claims

1. Nanoparticles **characterised in that** they comprise at least one polymer, at least one active constituent and at least one cyclic oligosaccharide.

2. Nanoparticles according to claim 1, **characterised in that** at least one of the polymers is a poly(alkyl cyanoacrylate) in which the linear or ramified alkyl group comprises 1 to 12 carbon atoms.

3. Nanoparticles according to either of claims 1 or 2, **characterised in that** the cyclic oligosaccharide is a neutral or charged, native, branched or polymerised or chemically modified cyclodextrine.

4. Nanoparticles according to any one of the previous claims, **characterised in that** the cyclic oligosaccharide is a cyclodextrine chemically modified by substitution of one or more hydroxypropyls by alkyl, aryl, arylalkyl, glycosidic groups, or be etherification or esterification with alcohols or aliphatic acids.

5. Nanoparticles according to any one of the previous claims, **characterised in that** the size of the nanoparticles is between 300 and less than 50 nm.

6. Nanoparticles according to any one of the previous claims, **characterised in that** the active constituent is hydrophilic, hydrophobic, amphiphilic and / or insoluble.

7. Nanoparticles according to any one of the previous claims, **characterised in that** the active constituent is chosen from among anti-cancer agents, antisense molecules, antivirals, antibiotics, proteins, polypeptides, polynucleotides, vaccinating substances, immunomodulators, steroids, analgesics, antimorphine, antifungal and antiparasite agents.

8. Nanoparticles according to claim 7, **characterised in that** the active constituent is taxol or one of its derivatives.

9. Nanoparticles according to claim 7, **characterised in that** the active constituent is doxorubicine or one of its derivatives.

10. Nanoparticles according to claim 7, **characterised in that** the active constituent is a derivative of platinum.

11. Nanoparticles according to any one of the previous claims, **characterised in that** the quantity of active constituent present is between 0.01 and 300 mg/g of nanoparticles.

12. Nanoparticles according to any one of the previous claims, **characterised in that** the proportion of cyclic oligosaccharide is between 0.1 and 70% by weight.

13. Method for preparation of nanoparticles based on a polymer according to any one of claims 1 to 12, **characterised in that** it comprises steps consisting of:
a) preparing a complex of at least one active constituent with at least one cyclic oligosaccharide in solution into an aqueous or non-aqueous solvent,
b) progressively adding at least one monomer of the polymer in the solution obtained in step (a), and
c) anionic or inducible polymerisation of this monomer, for example by other agents and particularly photochemical agents, possibly in the presence of one or more surfactants and / or stabilisers.

14. Process for preparation of nanoparticles based on a polymer according to any one of claims 1 to 12, **characterised in that** it comprises steps consisting of:
- preparing nanoparticles based on a polymer and more particularly poly(alkyl cyanoacrylates) and a cyclic oligosaccharide,
- associating the active constituent with the said nanoparticles.

15. Process for preparation of nanoparticles based on a polymer according to claim 14, **characterised in that** it comprises steps consisting of:
a) preparing a solution of at least one cyclic oligosaccharide in an aqueous or non-aqueous solvent,
b) progressively adding at least one monomer of the polymer, and more particularly the alkyl cyanoacrylate monomer into the solution obtained in step (a), and
c) polymerising this monomer, preferably by anionic polymerisation or possibly inducible polymerisation by other agents and particularly photochemical agents, possibly in the presence of one or more surfactants and / or stabilisers.
d) after an inspection and purification of the nanoparticles obtained in step (c) if necessary, incubate the said particles in a solution of the active constituent in an aqueous or non-aqueous solvent.

16. Process for preparation of nanoparticles based on a poly(alkyl cyanoacrylate) according to one of claims 13 to 15, **characterised in that** at least one alkyl cyanoacrylate monomer is added.

17. Process according to any one of claims 13 to 16, **characterised in that** the solvent used in steps (a), (b) and (d) is advantageously chosen so as to maximise the solubility of the active constituent and the cyclic oligosaccharide in the medium defined by this solvent, while maintaining conditions favourable to polymerisation of polymers and more particularly poly (alkyl cyanoacrylates).

18. Process according to one of claims 13 to 17, **characterised in that** step (c) is performed with no surfactant and / or stabiliser.

19. Process according to one of claims 13 to 17, **characterised in that** the proportion of cyclic oligosaccharide in step (a) is generally between 0.1 and 70% by weight of the active constituent.

20. Method according to one of claims 13 to 19, to manufacture a targeted medicine with an improved therapeutic index.

21. Nanoparticles including the association of a polymer and more particularly poly(alkyl cyanoacrylates) and a cyclic oligosaccharide, that can be obtained following steps (a) to (c) in a process according to claim 15.

22. Nanoparticles according to claim 21, **characterised in that** the cyclic oligosaccharide is a neutral or charged, native, branched or chemically modified cyclodextrine.
